# EUROPEAN PATENT SPECIFICATION

(11) **EP 0 701 699 B1**
(45) Date of publication and mention of the grant of the patent: **14.05.1997**
(21) Application number: 94917825.5
(22) Date of filing: 03.06.1994
(51) Int. Cl.: G01N 33/561, G01N 27/447

(54) **IMMUNOELECTROPHORESIS METHOD**
IMMUNOELEKTROFORESEVERFAHREN
PROCEDE D'IMMUNOELECTROPHORESE

(30) Priority: 04.06.1993 NL 9300965
(43) Date of publication of application: 20.03.1996
(73) Proprietor: BUSINESS INCEPTION B.V., 6721 AH Otterlo (NL)
(72) Inventor: MOEN, Alexander, Adrianus, NL-1622 LR Hoorn (NL); VAN VOORST, Jan, Johannes, NL-6731 AH Otterlo (NL)
(74) Representative: de Bruijn, Leendert C.
(86) International application number: NL9400129
(87) International publication number: WO9429726

(56) References cited:
- EP-A- 0 504 810
- DE-A- 3 811 083
- US-A- 5 228 960
- PLANTA MEDICA, vol.59, June 1993 pages 221 - 228 J.BRANDYS ET AL. 'Cross-Reactivity Between Pollen Extracts from Six Artemisia Species'
- JOURNAL OF CLINICAL CHEMISTRY AND CLINICAL BIOCHEMISTRY, vol.21, 1983 pages 841 - 844 G.MERLINI ET AL. 'Identification of Specific plasma Proteins determining the Agarose Gel Electrophoresis by the Immunosubtraction Technique.'
- JOURNAL OF DAIRY RESEARCH, vol.59, 1992 pages 273 - 285 L.K.RANTAMÄKI ET AL. 'Isolation and characterization of alpha2-macroglobulin from mastitis milk'

## Description

The present invention relates to a method of detecting in a test subject the presence of at least one hapten, one macromolecule, one pathogen or one part thereof that causes an immune response, as well as a method of detecting any immune response to the hapten, the macromolecule, the pathogen or at least one part thereof that causes an immune response.

The invention also relates to the use of said method for screening test subjects for contamination with at least one pathogen or antigen, for screening for any infection with at least one pathogen and/or for screening for an allergy to at least one hapten or antigen.

The invention also relates to the use of the method for distinguishing contamination with a wild-type pathogen from that with modified pathogens, as well as for distinguishing any immune response after contamination with a wild-type pathogen from an immune response after contact with a modified vaccine.

The method used most frequently at present for detecting specific antibodies to an antigen is ELISA (enzyme-linked immunosorbent assay). In ELISA, an antibody-antigen complex is detected by means of enzymatic colour reaction.

Other frequently used techniques for detecting antigen-specific antibodies are RIA (radioimmunoassay) and IRMA (immuno radiometric assay), where an antibody-antigen complex is detected by using radioactive labelling agents.

The greatest disadvantages associated with work involving radioactivity are the costs and the risk of contamination of the environment and the individual carrying out the method.

ELISA is not associated with the disadvantages mentioned in connection with radioactivity, and is more advantageous than RIA and IRMA in that the test is very sensitive and the costs when processing large numbers of samples are much lower. However, ELISA also has a number of disadvantages. ELISA can give many false-positive and false-negative assays because of the large number of vigorous washing steps required. There is only a relatively narrow margin within which the washing steps do not lead to false results; gentle washing always leads to false-positive readings, while vigorous washing removes immune complexes that have formed, so that they are not detected and false-negative results are obtained. The last-mentioned aspect occurs more frequently as the immune complexes become larger, for example in bacterial determinations where the complete microorganism is complexed. Furthermore, the ELISA technique requires many steps and is therefore fairly labour-intensive. In addition, ELISA indirectly reveals the presence of an immune complex by means of an enzymatic reaction. Moreover, it is not possible to detect an immune response of a test subject to pathogen A by the ELISA if an ELISA is being carried out with the aim of detecting any immune response to pathogen B. The simultaneous differentiation of disorders cannot be carried out effectively with ELISA techniques. However, it is possible, for example, to coat the wells in the microtiter plate used for an ELISA with a number of antigens specific to different pathogens, so that a number of antibodies characteristic of different infections with pathogens can be bound possibly from the sample, but in this case the sensitivity of the assay decreases considerably. Moreover, in this case it is only possible to demonstrate that the test subject shows an immune response to one of the pathogens, but it is impossible to determine to which pathogen.

There is a need for a direct method of determining the antigens against which the test subject has formed antibodies, in particular in the case of infection with proteins characteristic of pathogens. For this purpose, proteins in a sample of a test subject will have to be separated from one another and/or immunocomplexes will have to be separated from the other non-complexed proteins.

Electrophoresis is a frequently used technique for separating proteins in general. There are various types of electrophoresis, for example SDS gel electrophoresis, native gel electrophoresis and isoelectric focusing (IEF). In SDS gel electrophoresis the proteins are denatured; this is not the case with the last-mentioned two types. In the first two methods separation is effected via the size of the protein and in the last-mentioned type this is done on the basis of the isoelectric point.

In view of the fact that antibodies electrophorese at the same point due to their large correspondence in terms of size, charge and mass and in view of the fact that direct detection of specific antibodies to a specific antigen in situ on the gel is impossible, because it is not possible to introduce the antigen in the gel at the same time as keeping the antibodies in place, the use of just electrophoresis for the specific detection of a certain antigen or antibody in a sample transferred to gel is impossible. Due to the fact that the component of the immune complex to be applied has to diffuse through the gel in order to reach the components of the sample and, if appropriate, to react therewith, and that of course the components of the sample also diffuse in the meantime, this makes it impossible to develop a reliable detection pattern.

In order to solve this problem when applying electrophoresis in immunochemistry, the blotting techniques were developed. In these blotting techniques, protein-binding paper is brought into contact with a gel on which proteins have been separated. By application of pressure or electrophoresis, the proteins are subsequently transferred on to the paper, a fixed impression of the protein components being formed on the paper as they have separated on the gel. Specific detectable immune complexes can subsequently be formed in the same way as in ELISA and RIA by application of a specific antibody directed against the pathogen, part of a pathogen, hapten or protein to be detected, or an impression of the antibodies can be made on paper and the specific antigen can then be brought into contact with the latter and the immune complex which may result can then be rendered detectable. The blotting techniques also have disadvantages. The technique is slow, expensive, inefficient and insensitive. On blotting, the copying of the gel on to the paper, only part of the separated sample constituents is transferred on to the paper, so that the test is inefficient. In addition, the disadvantages reported for ELISA and RIA above apply in the same way.

The existing techniques are insufficient for direct determination of the antigens against which the test subject has formed antibodies. There is no test which shows in an efficient and simple manner that a test subject is contaminated and possibly infected and at the same time shows what has contaminated and possibly infected the test subject.

In J. Clin. Chem.Clin. Biochem. Vol. 21, 1983, pp 841-844 Merlini G. et al. describe use of the immunosubtraction technique to identify the proteins in electrophoretically separated plasma. The technique consists of forcing the plasma through a layer of monospecific antibody purified by electrophoresis, thereby precipating the specific protein which is consequently absent from the subsequent electrophoretogram. The immunosubtraction technique consists of two steps:
1) electrophoretic purification of the gamma fraction of the antiserum and
2) electrophoresis of the sample through the purified antibodies. Antibody was applied with a microsyringe to an agarose gel and antisera were applied in alternate wells, leaving the adjacent wells free for the control, i.e. non-immunosubtracted, sera. Once absorbed, the antisera were subjected to electrophoresis at 20 V/cm for 45 min.. After this preliminary electrophoresis a strip of agarose gel, 1,5 cm wide, was removed from the deposition side of the plate because it is contaminated with the non-gamma fractions of the antisera and replaced with a new strip of the same gel. Subsequently the samples diluted with saline containing bromophenol-blue were placed both in the wells previously filled with the antisera, and in the adjacent wells which had been left free for comparative analysis. Immunosubtracted and non-immunosubtracted samples therefore migrated in parallel. The second electrophoretic run was carried out with inverted polarity then the samples were electrophoresed at 10 V/cm for the first 15 min. and at 20 V/cm for the following 30-40 min.. The current intensity was kept initially lower in order to facilitate the antigen antibody reaction. The disadvantages of this technique are numerous. With this technique it is impossible to detect antigens that migrate slower than the gammaglobulins because this would mean the antigens would never be able to overtake the gammaglobulins and form an immunocomplex with them. Also it is impossible to detect IgM with the method described by the Italians. It is only possible to detect IgG of the immunoglobulins. All the non-gamma fractions of sera have been cut away in the method according to Merlini et al.. The method according to Merlini et al. has the further disadvantages that two electrophoresis steps are required and that the electrophoresis must be carried out slowly, otherwise the antigen will run past the immunoglobulin without reacting with it and thereby causing false negative tests results.

The method according to Merlini et al. is dependent on the migration speed of the substance which has to be detected in the sample. This substance must run faster through the gel than the binding partner that has been previously incorporated in the gel. Therefore, the method according to Merlini et al. requires a pre-analysis of which material runs faster in order to determine whether it can be detected or not. In particular, if one were interested in determining the presence of a specific pathogen such as a virus in a sample and one were to destroy the virus then the virus fragments would probably not migrate faster than the IgG fraction therefore be undetectable as causing immunocomplexes. In particular when one is interested in detecting an infection at an early stage one is interested in detecting the presence of IgM as this is produced in first instance of an infection whereas the IgG production at the beginning of the infection is very small and often not detectable. Therefore, the method of Metlini et al. could only be used to determine infection at a late stage of infection, in contrast to the subject method, which is capable of detecting IgM at an early stage of infection.

In the US patent 5 228 960 published after the filing of the priority patent application of the subject invention, i.e. published on July 20, 1993 but filed prior to the priority application of that subject invention, i.e. July 17, 1992 corresponding to EP-A-0 579 361 [cited under Art 54(3) EPC] a methodology is disclosed for capillary electrophoretic immunosubtraction of samples as a means of providing analysis of monoclonal gammopathies. In particular, the method disclosed is a capillary electrophoretic immunosubtraction method. Preferably of a sample comprising constituent parts to be separated, said method comprising the step of
1) separating a first aliquot of the sample into constituent parts by capillary electrophoretic techniques and detecting said parts,
2) admixing a second aliquot of said sample with at least one specific binding partner to a constituent of said sample, said specific binding partner capable of being substantially removed from said aliquot;
3) separating said aliquot into constituent parts by capillary electrophoretic techniques and detecting said parts; and
4) comparing the separated constituent parts of step 3) with the separated constituent parts of step 1).

Preferably, the sample is a clinical sample and the constituent parts include immunoglobulins whereby the specific binding partner is an anti-immunoglobulin antibody. Most preferably, the specific binding partner is either insolubilized before the second aliquot is admixed therewith, or is capable of being insolubilized after admixture with the aliquot.

In the method described in the U.S. Patent it is necessary to immunoprecipitate or remove any immunocomplexes from the sample as otherwise the capillaries will quickly be blocked by the large immunocomplexes.

EP-A-0 504 810 relates to a specific assay of body fluids for detection of the presence of glyco-conjugates having an isoelectrical point between pH 7 and 10. In the preferred embodiment the total protein content of the serum are separated by isoelectric focusing (IEF) in a pH range of 6,5 to 9,5. The separated proteins are transferred to a membrane, whereafter the carbohydrate containing compounds are indicated immunologically (see the abstract and page 3, last paragraph).

DE-A-3 811 083 refers to an immunoassay for the detection of an antigen, which is electrically neutral. The antigen is reacted with an electrically charged antibody. The reaction product is thereafter treated electrophoretically followed by the quantitative determination of the antigen in question.

In Journal of Diary Research, Vol. 59, 1992, pages 273-285 L.K. Rantamäti et al. describe isolation and characterization of α₂-macroglobulin from mastitis milk. The components of whey samples from mastitis milk were analysed by SDS-Page and native-PAGE technique.

Surprisingly, a method has now been found with which the antigens against which a test subject, an experimental animal or a human being has formed antibodies can be determined directly by means of electrophoresis.

The present invention relates to a method of detecting in a test subject the possible presence of at least one hapten, macromolecule, pathogen or one part thereof that causes an immune response, and/or of detecting any immune response to at least the hapten, macromolecule, pathogen or one part thereof that causes an immune response, in which
a) a liquid sample of the test subject to be tested is taken or prepared,
b) the sample is incubated with a stock solution, which stock solution comprises at least one characteristic antigen of the pathogen to be tested, of one part of the pathogen to be tested, of the macromolecule to be tested or of the hapten to be tested, or which stock solution comprises at least one antibody which is specific to the pathogen to be tested, for a part of the pathogen to be tested, for the macromolecule to be tested or for the hapten to be tested,
c) the incubated mixture is applied, after incubation, to a substrate suitable electrophoresis under non-denaturing conditions,
d) the same stock solution that was used in step b) is applied to the same substrate as reference,
e) the mixture of step c) and the stock solution of step d) are subjected to electrophoresis under non-denaturing conditions, the components of the incubation mixture and the stock solution being separated,
f) the components of the incubation mixture and the stock solution are rendered detectable in one of the above-mentioned steps, and
g) the signal patterns resulting after the electrophoresis of the incubation mixture and the stock solution are compared. The difference after electrophoresis between stock solution and sample due to the possible formation of immune complexes is analyzed.

During the incubation step of the liquid sample with the stock solution, which stock solution comprises, for example, at least one specific antigen to be detected, any antibodies present in the sample which are specific to the antigen present in the stock solution can react to form the immune complex with their corresponding antigen. The method according to the invention can be used for detecting the presence of any type of immunoglobulin such as IgG, IgM, IgE, IgA. In particular the possibility of IgM detection is advantageous as early stages of infection can thus be positively diagnosed as infection. When the test subject has shown an immune response as reaction to contamination or inoculation with the specific antigen to be detected, the corresponding antibody specific to that antigen will be present in the serum sample of the test subject. The antibody will bind to the antigen present in the stock solution. Immune complexes will be formed. By forming the immune complex, certain molecular parameters of the antigen thus bound will change in relation to unbound antigen. This change in parameters, for example the isoelectric point and the molecular weight, can be observed in electrophoresis when the stock solution is employed as a reference in the electrophoresis in addition to the incubation mixture. Since it is intended with this method to monitor the complexes of proteins, in particular, of antibody-antigen, it is necessary to carry out the electrophoresis under non-denaturing conditions. Surprisingly, it has been found that the immune complexes formed do not denature or dissociate under the influence of gel constituents such as glycerol or ampholines and that the intended object can therefore be achieved. An expert in the field of proteins knows which non-denaturing conditions are commonly used for protein separation in gel electrophoresis and can apply them readily when carrying out the method according to the invention.

Although SDS-PAGE gives the best results for the blotting technique, it is unsuitable for the intended object because denaturation of proteins takes place. Native PAGE and IEF can be used in the method according to the subject invention. There are however noticeable differences between both techniques. The separation capability of IEF is much larger than the separation capability of native PAGE. Native PAGE electrophoresis is possible at a fixed pH, which is not possible with IEF. When using the subject invention one or the other of the techniques will be preferred. A person skilled in the art will appreciate which technique is preferred in which circumstances. Capillary electrophoresis is not useful in the subject method as immunocomplexes will block the capillaries. In all cases as the formed immunocomplex must remain in tact denaturing circumstances must be avoided.

Noticeably the possible formation of immune complexes occurs prior to electrophoresis so that the reaction partners do not have to find eachother in the gel in contrast to existing methods, thereby eliminating a possible source of incorrect results such as occurs in the method of Merlini et al.. Prior to the subject invention a person skilled in the art would not have considered reacting the sample with a binding partner prior to electrophoresis as the resulting immunocomplexes were considered to be too large to enter a gel. Surprisingly it was found that a 4% acrylamide gel for example is quite capable of electrophoresis of immunocomplexes which have a size in the order of 500-600,000 Daltons. In particular it is even possible to detect situations where multiple antigens bind to antibody and also where multiple antibodies bind to an antigen. The general size of an immunoglobulin is 160,000 Daltons. In particular the difference to previous techniques is that in the current method the absence or presence of an immune complex is assessed whereas previously the immunocomplexes were discarded.

The advantage of the present invention resides in the fact that the multiple washing steps and manipulations of the sample usually required with ELISA and the like are unnecessary. There are no false-positive or false-negative readings and the present method can also be carried out for large numbers of samples due to the easy procedure. Further advantage of the subject method lies in the fact that any type of antibody can be detected, not only an IgG but also an IgM or an IgA. It is also possible to use the method on samples comprising a little antibody and using excess antigen or for samples comprising a lot of antibody with addition of a little antigen. It is also possible to use the subject method when there is an equivalent amount of antigen to antibody. In short therefore the dosage of the amount of a specific binding partner is not relevant to the outcome of the test. All variants will give the desired results. Furthermore, the electrophoresis can occur in one simple step thereby simplifying the detection and thereby also lowering the costs. Furthermore, the technique no longer requires that the antigens have to be faster migratory elements than the specific binding partner. Nor is it necessary to carry out electrophoresis slowly in order to prevent false negatives as the reaction between this specific binding partner and the element to be detected occurs prior to electrophoresis thereby rendering the method independent of the voltage applied. In the method described by Merlini et al. a low voltage is necessary to avoid the false negatives. It is also possible with the subject method to detect early stages of infection in which generally speaking immunoglobulin G is produced in a very low amount and IgM is produced in a large amount, because the subject method allows detection also of IgM.

It is possible with the present invention to screen for specific macromolecules or haptens, and not only for an "all-over" pathogen. A macromolecule can be understood to mean, inter alia, a protein or a polypeptide. Depending on the composition of the stock solution, it is also possible to screen for more than one disorder in a single operation.

At the same time, it is possible with the method according to the invention to use precisely that pH range which is of interest with respect to a specific disorder. If IEF is employed as the electrophoresis step, electrophoresis can be carried out, for example, in a pH range of between 3 and 10 but, if screening is carried out for a specific contamination whose characteristic antigen electrophoreses at a pH of 6-7, electrophoresis can be carried out just within this pH range, so that a specific result can be obtained more quickly and more unambiguously.

All the immune complexes and protein components of the gel can be rendered detectable by employing the usual colour reactions for proteins. The method according to the invention can also be carried out in such a way that each characteristic antigen or specific antibody of the stock solution in step b) is provided with a labelling agent. In this way, it is also possible to label different antigens or antibodies of the stock solution separately or in groups with different labels depending on what is to be detected.

The present invention also relates to the use of the method according to the invention, which method is called electrophoretic profiling for the sake of clarity, for screening of a test subject for the presence of at least one pathogen or part thereof, or for screening of a test subject for an immune response in the case of infection or inoculation with at least one pathogen or part thereof.

When used in this way, detection of a displacement and/or disappearance of at least one signal of the signal pattern of the incubation mixture in comparison with that of the reference after electrophoresis indicates contamination and possibly also infection with the applied pathogen, part of the pathogen that causes an immune response, macromolecule or hapten employed. The signal can be in the form of one or more bands or stripings on the gel or the peak of an electrophoretogram or can be the occurrance or absence of a large blotch or peak near or at the application site of the sample in the gel. In order to detect contamination with a specific pathogen, it is necessary to use the pathogen itself in the stock solution or to use at least one antigen characteristic of infection with the pathogen in the stock solution. If contamination with a number of different pathogens is to be detected by screening, the stock solution should comprise the corresponding pathogens or antigens thereof, which antigens lead to an immune response characteristic of an infection.

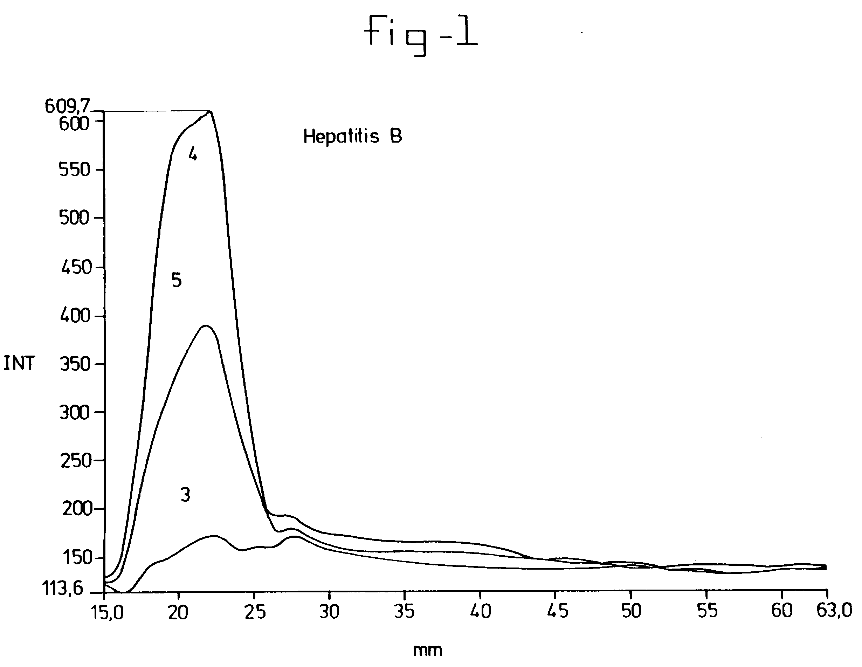

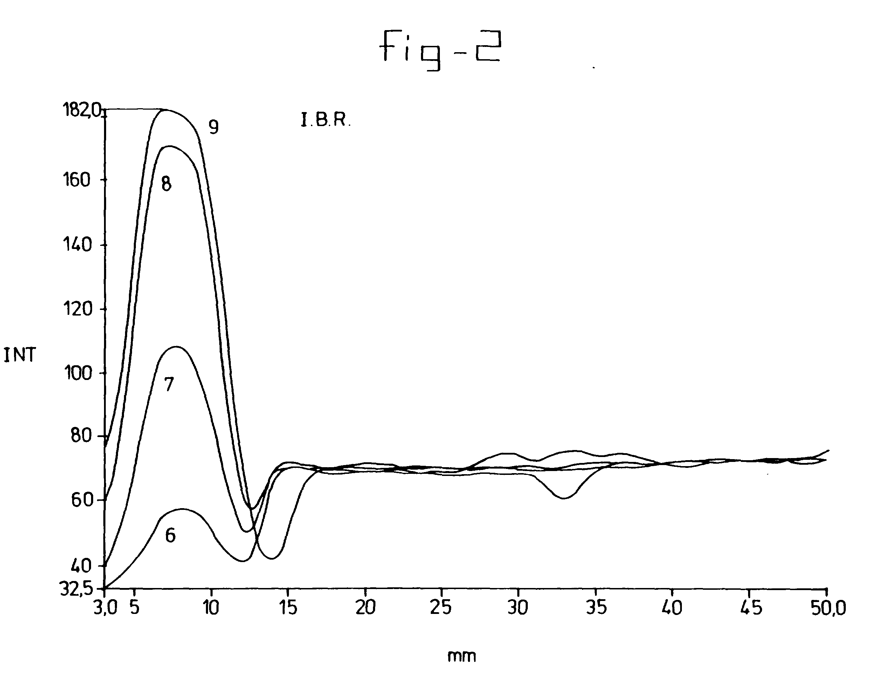

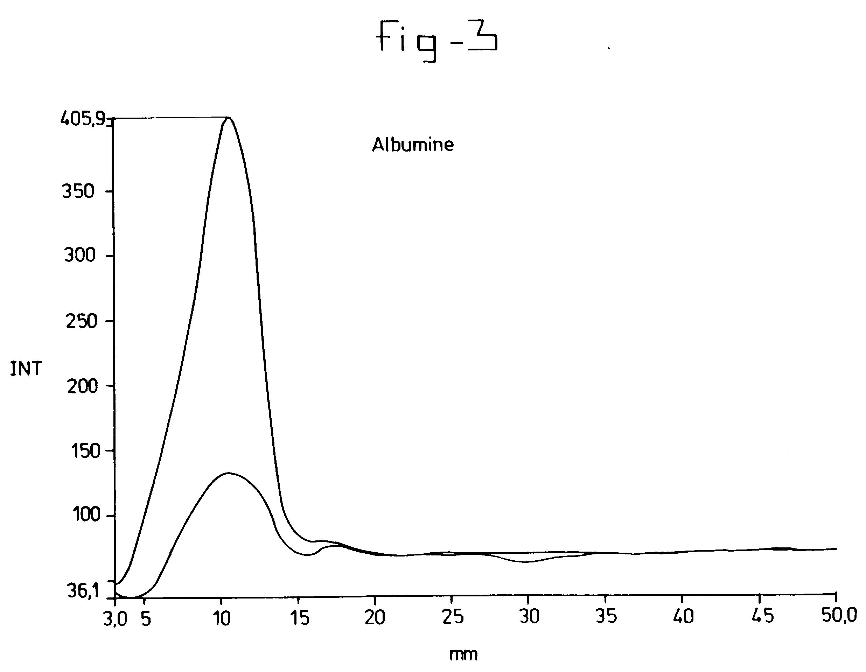

However, if screening is used only to detect a contamination but not an immune response, it is possible to use a specific antibody for the pathogen to be detected in the stock solution and subsequently to monitor whether any complex formation has taken place in the incubation mixture according to the present electrophoretic profiling technique. Such complex formation can only take place if the antigen to be detected is present in the sample. Figure 1 illustrates use of the method according to the invention, i.e. electrophoretic profiling to determine infection with hepatitis B. The test was carried out in a 12% gel at 1500 Vh. Peak 3 is without antiserum, 4 with antiserum and 5 with 10x diluted antiserum. Figure 2 illustrates use of electrophoretic profiling to determine infection with IBR in a 4% gel at 1500 Vh. Peak 6 is without antigen. Peak 7 is with cow 1, peak 8 with cow 2 and peak 9 with cow 3. Quite clearly cows 1-3 are infected. Figure 3 illustrates the determination of the presence of antibodies to albumin using the method according to the invention on a 4% gel. In all figures the intensity of the band is given vertically and the location on the gel is expressed horizontally in mm.

The invention also covers the use of the electrophoretic profiling technique according to the invention for screening for an allergy to at least one hapten or antigen, in which detection of complex formation in the incubation mixture according to the subject technique of electrophoretic profiling in comparison with that of the reference after electrophoresis indicates an allergy to the hapten or allergen employed.

Another use of the present electrophoretic profiling technique, which is also interesting, is the use for distinguishing between contamination with a wild-type pathogen and immunisation with a modified vaccine, in which displacement and/or disappearance of signals with concomitant appearance of immunocomplexes in the gel or application site of the sample on the gel of all proteins characteristic for infection with wild-type pathogen indicates that an infection with a wild-type pathogen has taken place, and wherein the presence of at least one antigen, characteristic of an immune response and/or the absence of at least one antigen characteristic for immune reaction in the formed immunocomplexes, indicates that no antibodies have been formed against at least one antigen determining the immune response of the pathogen, which indicates that the immune response of the test subject is not directed against the wild-type pathogen but against the modified vaccine. In the present case, a modified vaccine is understood to mean a vaccine where either at least one protein is missing which causes a characteristic immune response on contamination with the wild-type pathogen, or a vaccine which comprises at least one protein mutated in such a way that binding with the antibody produced against the corresponding wild-type protein during infections no longer occurs.

Using the electrophoretic profiling technique according to the invention, it is possible
- to detect both the presence and absence of pathogens,
- to detect the response of the body of a test subject to pathogens, parts of pathogens, antigens or haptens, as desired, and
- to determine the state of health of the test subject as regards the chosen pathogens, parts of pathogens, proteins, haptens or antigens.

The technique can be generally employed for diagnosing proteins present in body fluids of humans and animals. Such body fluids may be serum, blood, blood cells, urine, breast milk and saliva.

The method according to the invention can be applied more specifically for
- eradication programmes;
- demonstrating differences between natural immunity and immunity as a result of modified vaccines;
- the preventative screening of populations, including company populations such as farm animals, for the presence of pathogens or the occurrence of immune responses to pathogens.

## Claims

1. Method of detecting the possible presence of at least one hapten, one macromolecule, one pathogen or one part thereof that causes an immune response in a test subject, and/or of detecting an immune response to at least one hapten, one macromolecule, one pathogen or one part thereof that causes an immune response, in which
a) a liquid sample of the test subject to be tested is taken or prepared,
b) the sample is incubated with a stock solution, which stock solution comprises at least one characteristic antigen of the pathogen of which the immune response is to be tested, of one part of the pathogen of which the immune response is to be tested, of the macromolecule of which the immune response is to be tested or of the hapten of which the immune response to be tested, or which stock solution comprises at least one antibody which is specific to the pathogen to be tested, for one part of the pathogen to be tested, for the macromolecule to be tested or for the hapten to be tested,
c) the incubated mixture is applied, after incubation, to a substrate suitable for electrophoresis under non-denaturing conditions,
d) the same stock solution that was used in step b) is applied to the same substrate as reference,
e) the mixture of step c) and the stock solution of step d) are subjected to electrophoresis under non denaturing conditions, the components of the incubation mixture and the stock solution being separated,
f) the components of the incubation mixture and the stock solution are rendered detectable in one of the above-mentioned steps, and
g) the signal patterns resulting after the electrophoresis of the incubation mixture and the stock solution in particular with regard to proteins and immune complexes are compared.

2. Method according to Claim 1, in which each characteristic antigen or specific antibody of the stock solution of b) is provided with a labelling agent.

3. Method according to Claim 1 or 2, in which native PAGE is carried out in step e).

4. Method according to Claim 1 or 2, in which IEF is carried out in step e).

5. Method according to one of the preceding claims, in which the stock solution comprises at least the pathogen itself.

6. Use of a method according to one of the preceding claims for screening for the presence of or contamination and possibly also infection with at least one pathogen, in which detection of a displacement and/or disappearance of at least one signal of the signal pattern of the incubation mixture in comparison with that of the reference after electrophoresis in particular detection of a displacement and/or disappearance of at least one band or peak of the band or peak pattern and/or appearance of one or more bands or stripings in the gel due to the presence of immunocomplexes in the gel and/or presence of immune complexes at the application site indicates contamination with the pathogen employed.

7. Use of a method according to one of Claims 1-4 for screening for an allergy to at least one hapten or antigen, in which detection of a displacement of at least one signal of the signal pattern of the incubation mixture in comparison with that of the reference after electrophoresis in particular detection of a displacement and/or disappearance of at least one band or peak of the band or peak pattern and/or appearance of one or more bands or stripings in the gel due to the presence of immune complexes in the gel and/or presence of immune complexes at the application site indicates an allergy to the hapten or allergen employed.

8. Use of a method according to one or more of Claims 1-5, for distinguishing between infection with a wild-type pathogen and immunisation with a modified vaccine, in which a displacement and/or disappearance of signals of the signal pattern, e.g. bands and simultaneous appearance of immune complexes in the gel or application site of all proteins characteristic for infection with wild-type pathogen indicates that infection with wild-type pathogen has taken place and wherein the presence of at least one antigen characteristic of immune reaction and/or the absence of at least one antigen characteristic for immune response in the formed immune complexes indicates that no antibodies have been formed against at least one antigen characteristic for the immune response of the pathogen, which indicates that the test subject is not infected with the wild-type pathogen but has had an immune response after contact with a modified vaccine.

## Patentansprüche

1. Verfahren zum Nachweisen des möglichen Vorhandenseins mindestens eines Haptens, eines Makromoleküls, eines Pathogens oder eines Teils davon, das eine Immunantwort in einem Testsubjekt hervorruft,und/oder zum Nachweisen einer Immunantwort auf mindestens ein Hapten, ein Makromolekül, ein Pathogen oder ein Teil davon, das eine Immunanwort hervorruft, worin
a) eine flüssige Probe des zu untersuchenden Testsubjekts genommen oder hergestellt wird,
b) die Probe mit einer Stammlösung inkubiert wird, wobei diese Stammlösung mindestens ein charakteristisches Antigen des Pathogens, dessen Immunantwort untersucht werden soll, eines Teils des Pathogens, dessen Immunantwort untersucht werden soll, des Makromoleküls, dessen Immunantwort untersucht werden soll, oder des Haptens, dessen Immunantwort untersucht werden soll, umfaßt oder wobei die Stammlösung mindestens einen Antikörper umfaßt, der für das zu untersuchende Pathogen, für ein zu untersuchendes Teil des Pathogens, für das zu untersuchende Makromolekül oder für das zu untersuchende Hapten spezifisch ist,
c) die inkubierte Mischung nach der Inkubation auf ein für eine Elektrophorese unter nichtdenaturierenden Bedingungen geeignetes Substrat aufgetragen wird,
d) die gleiche Stammlösung, die in Schritt b) verwendet wurde, auf das gleiche Substrat als Referenz aufgetragen wird,
e) die Mischung von Schritt c) und die Stammlösung von Schritt d) einer Elektrophorese unter nichtdenaturierenden Bedingungen unterworfen werden, wobei die Bestandteile der Inkubationsmischung und der Stammlösung getrennt werden,
f) die Bestandteile der Inkubationsmischung und der Stammlösung in einem der obenerwähnten Schritte nachweisbar gemacht werden, und
g) die Signalmuster, welche sich nach der Elektrophorese der Inkubationsmischung und der Stammlösung ergeben, insbesondere im Hinblick auf Proteine und Immunkomplexe verglichen werden.

2. Verfahren nach Anspruch 1, wobei jedes charakteristische Antigen oder jeder spezifische Antikörper der Stammlösung von b) mit einem Markierungsmittel versehen wird.

3. Verfahren nach Anspruch 1 oder 2, wobei in Schritt e) eine native Polyacrylamidgelelektrophorese (PAGE) durchgeführt wird.

4. Verfahren nach Anspruch 1 oder 2, wobei in Schritt e) eine isoelektrische Fokussierung (IEF) durchgeführt wird.

5. Verfahren nach einem der vorhergehenden Ansprüche, wobei die Stammlösung mindestens das Pathogen selbst umfaßt.

6. Verwendung eines Verfahrens nach einem der vorhergehenden Ansprüche zum Screening auf das Vorhandensein von oder die Kontamination und möglicherweise auch Infektion mit mindestens einem Pathogen, wobei der Nachweis einer Verschiebung und/oder des Verschwindens von mindestens einem Signal des Signalmusters der Inkubationsmischung im Vergleich zu dem der Referenz nach der Elektrophorese, insbesondere der Nachweis einer Verschiebung und/oder des Verschwindens von mindestens einer Bande oder eines Peaks des Banden- oder Peakmusters und/oder das Auftreten von einer oder mehreren Banden oder Streifen im Gel aufgrund des Vorhandenseins von Immunkomplexen in dem Gel und/oder des Vorhandenseins von Immunkomplexen an der Auftragungsstelle eine Kontamination mit dem eingesetzten Pathogen anzeigt.

7. Verwendung eines Verfahrens nach einem der Ansprüche 1 bis 4 zum Screening auf eine Allergie gegen mindestens ein Hapten oder Antigen, wobei der Nachweis einer Verschiebung von mindestens einem Signal des Signalmusters der Inkubationsmischung im Vergleich zu dem der Referenz nach der Elektrophorese, insbesondere der Nachweis einer Verschiebung und/oder des Verschwindens von mindestens einer Bande oder eines Peaks des Banden- oder Peakmusters und/oder das Auftreten von einer oder mehreren Banden oder Streifen in dem Gel aufgrund des Vorhandenseins von Immunkomplexen in dem Gel und /oder des Vorhandenseins von Immunkomplexen an der Auftragungsstelle eine Allergie gegen das eingesetzte Hapten oder Allergen anzeigt.

8. Verwendung eines Verfahrens nach einem oder mehreren der Ansprüche 1 bis 5 zum Unterscheiden zwischen einer Infektion mit einem Wildtyp-Pathogen und einer Immunisierung mit einem modifizierten Impfstoff, wobei die Verschiebung und/oder das Verschwinden von Signalen des Signalmusters, z.B. Banden, und das gleichzeitige Auftreten von Immunkomplexen in dem Gel oder an der Auftragungsstelle von allen Proteinen, die für eine Infektion mit dem Wildtyp-Pathogen charakteristisch sind, anzeigt, daß eine Infektion mit Wildtyp-Pathogen stattgefunden hat, und wobei das Vorhandensein von mindestens einem für eine Immunreaktion charakteristischen Antigen und/oder die Abwesenheit von mindestens einem für die Immunantwort charakteristischen Antigen in den gebildeten Immunkomplexen anzeigt, das keine Antikörper gegen mindestens ein für die Immunantwort des Pathogens charakteristisches Antigen gebildet worden sind, was anzeigt, daß das Testsubjekt nicht mit dem Wildtyp-Pathogen infiziert ist sondern eine Immunantwort nach dem Kontakt mit einem modifzierten Impfstoff gehabt hat.

## Revendications

1. Méthode de détection de la présence éventuelle d'au moins un haptène, une macromolécule, un agent pathogène ou une partie de celui-ci qui provoque une réponse immunitaire chez un sujet test, et/ou de détection d'une réponse immunitaire à au moins un haptène, une macromolécule, un agent pathogène ou une partie de celui-ci qui provoque une réponse immunitaire, méthode dans laquelle
a) on prélève ou on prépare un échantillon liquide à tester du sujet test,
b) on incube l'échantillon avec une solution stock, solution stock qui comprend au moins un antigène caractéristique de l'agent pathogène dont on doit tester la réponse immunitaire, d'une partie de l'agent pathogène dont on doit tester la réponse immunitaire, de la macromolécule dont on doit tester la réponse immunitaire ou de l'haptène dont on doit tester la réponse immunitaire, ou solution stock qui comprend au moins un anticorps qui est spécifique de l'agent pathogène à tester, pour une partie de l'agent pathogène à tester, pour la macromolécule à tester ou pour l'haptène à tester,
c) on dépose le mélange incubé, après incubation, sur un substrat approprié pour électrophorèse dans des conditions non dénaturantes,
d) on dépose la même solution stock que celle utilisée à l'étape b) sur le même substrat comme témoin,
e) on soumet le mélange de l'étape c) et la solution stock de l'étape d) à une électrophorèse dans des conditions non dénaturantes, les constituants du mélange d'incubation et de la solution stock étant séparés,
f) on rend les constituants du mélange d'incubation et de la solution stock détectables dans l'une des étapes mentionnées ci-dessus, et
g) on compare les profils de signaux obtenus après l'électrophorèse du mélange d'incubation et de la solution stock en particulier en ce qui concerne les protéines et les complexes immuns.

2. Méthode selon la revendication 1, dans laquelle chaque antigène caractéristique ou anticorps spécifique de la solution stock de b) est fourni avec un agent de marquage.

3. Méthode selon la revendication 1 ou 2, dans laquelle on effectue une électrophorèse sur gel de polyacrylamide (PAGE) native à l'étape e).

4. Méthode selon la revendication 1 ou 2, dans laquelle on effectue une focalisation isoélectrique (IEF) à l'étape e).

5. Méthode selon l'une des revendications précédentes, dans laquelle la solution stock comprend au moins l'agent pathogène lui-même.

6. Utilisation d'une méthode selon l'une des revendications précédentes pour rechercher la présence d'au moins un agent pathogène ou une contamination et éventuellement également une infection par au moins un agent pathogène, dans laquelle la détection d'un déplacement et/ou de la disparition d'au moins un signal du profil de signaux du mélange d'incubation en comparaison avec celui du témoin après électrophorèse indique une contamination par l'agent pathogène employé, et en particulier la détection d'un déplacement et/ou de la disparition d'au moins une bande ou un pic de la bande ou un profil de pics et/ou l'apparition d'une ou plusieurs bandes ou série de bandes dans le gel due à la présence d'immunocomplexes dans le gel et/ou à la présence de complexes immuns au site de dépôt.

7. Utilisation d'une méthode selon l'une des revendications 1-4 pour rechercher une allergie à au moins un haptène ou un antigène, dans laquelle la détection d'un déplacement d'au moins un signal du profil de signaux du mélange d'incubation en comparaison avec celui du témoin après électrophorèse indique une allergie à l'haptène ou à l'allergène employé et en particulier la détection d'un déplacement et/ou de la disparition d'au moins une bande ou un pic de la bande ou un profil de pics et/ou l'apparition d'une ou plusieurs bandes ou série de bandes dans le gel due à la présence de complexes immuns dans le gel et/ou à la présence de complexes immuns au site de dépôt.

8. Utilisation d'une méthode selon l'une au moins des revendications 1-5, pour faire la distinction entre une infection par un agent pathogène de type sauvage et une immunisation avec un vaccin modifié, dans laquelle un déplacement et/ou la disparition de signaux du profil de signaux, par exemple, des bandes et une apparition simultanée de complexes immuns dans le gel ou au site de dépôt de toutes les protéines caractéristiques de l'infection par l'agent pathogène de type sauvage indique que l'infection par l'agent pathogène de type sauvage a eu lieu et dans laquelle la présence d'au moins un antigène caractéristique de la réaction immunitaire et/ou l'absence d'au moins un antigène caractéristique de la réponse immunitaire dans les complexes immuns formés indique qu'il n'y a pas d'anticorps formés contre au moins un antigène caractéristique de la réponse immunitaire de l'agent pathogène, ce qui indique que le sujet test n'est pas infecté par l'agent pathogène de type sauvage mais a eu une réponse immunitaire après contact avec un vaccin modifié.
